**Europäisches Patentamt**

(19) **European Patent Office**

**Office européen des brevets**

(11) **EP 1 280 834 B1**

(12) **EUROPÄISCHE PATENTSCHRIFT**

(45) Veröffentlichungstag und Bekanntmachung des Hinweises auf die Patenterteilung:
**19.05.2004 Patentblatt 2004/21**

(21) Anmeldenummer: **01964679.3**

(22) Anmeldetag: **07.03.2001**

(51) Int Cl.7: **C08F 8/44**, A61L 15/00

(86) Internationale Anmeldenummer:
**PCT/EP2001/002555**

(87) Internationale Veröffentlichungsnummer:
**WO 2001/074913 (11.10.2001 Gazette 2001/41)**

(54) **PULVERFÖRMIGE, AN DER OBERFLÄCHE VERNETZTE POLYMERISATE**

SURFACE CROSS-LINKED POWDER POLYMER

POLYMERES EN POUDRE RETICULES EN SURFACE

(84) Benannte Vertragsstaaten:
**AT BE CH CY DE DK ES FI FR GB GR IE IT LI LU MC NL PT SE TR**

(30) Priorität: **31.03.2000 DE 10016041**

(43) Veröffentlichungstag der Anmeldung:
**05.02.2003 Patentblatt 2003/06**

(73) Patentinhaber: **Stockhausen GmbH & Co. KG**
**47805 Krefeld (DE)**

(72) Erfinder:
• **INGER, Waldemar**
**47829 Krefeld (DE)**
• **HOSE, Rüdiger**
**47918 Tönisvorst (DE)**
• **BOHLMANN, Heinz-Peter**
**47800 Krefeld (DE)**

(74) Vertreter: **Herzog, Martin**
**Kahlhöfer . Neumann . Herzog . Fiesser,**
**Karlstrasse 76**
**40210 Düsseldorf (DE)**

(56) Entgegenhaltungen:
EP-A- 0 233 067      EP-A- 0 882 502
WO-A-96/05234      FR-A- 1 304 002
US-A- 5 145 906      US-A- 5 502 089

**Beschreibung**

Die vorliegende Erfindung betrifft ein pulverförmiges, an der Oberfläche nachvernetztes, Wasser oder wäßrige Flüssigkeiten absorbierendes Polymerisat, aufgebaut aus polymerisierten gegebenenfalls vorvernetzten, teilneutralisierten Carboxylgruppen enthaltenden Monomeren. Die vorliegende Erfindung betrifft außerdem ein Verfahren zur Nachbehandlung der o.g. Polymerisate und die Verwendung einer Lösung von mindestens einem dreiwertigen Kation zur Wiederherstellung der Gelpermeabilität der o.g. Polymerisate, die durch mechanische Einwirkung geschädigt worden sind.

Wässrige Flüssigkeiten absorbierende Polymerisate, sogenannte Superabsorber sind durch zahlreiche Veröffentlichungen bekannt. Es kommen modifizierte natürliche Polymere und teilweise oder vollständig synthetische Polymere zum Einsatz. Die vollsynthetischen Polymeren werden in der Regel durch radikalische Polymerisation verschiedener hydrophiler Monomere in wässriger Lösung nach unterschiedlichen Methoden hergestellt. Im allgemeinen werden dabei Vernetzer einpolymerisiert, wodurch das erhaltene Polymerisat nicht mehr wasserlöslich, sondern nur noch wasserquellbar ist. Als Superabsorber können z.B. Polymerisate auf Basis von (Meth-) Acrylsäure verwendet werden, die in teilweise neutralisierter Form als Alkalisalz vorliegen.

Das Superabsorberpolymer wird in der Regel nach der Polymerisation mechanisch zerkleinert, getrocknet und gemahlen. Dabei fällt das pulverförmige wasserquellbare Polymer je nach Herstellungsverfahren in einem mehr oder weniger breiten Kornspektrum an, das typischerweise in einem Bereich von 10 bis 1000 μm liegt, wovon üblicherweise die Komfraktion 150 bis 850 μm für praktische Zwecke als Absorptionsmaterial vor allem im Hygienesektor eingesetzt wird. Feinanteile < 150 μm sind aufgrund ihres Staubverhaltens und ihrer inhalationstoxischen Eigenschaften unerwünscht.

Die Entwicklung neuerer Windelkonstruktionen ist von dem Trend geprägt, immer größere Anteile des voluminösen Cellulosefluffs durch Superabsorber zu ersetzen. Dies geschieht aus Gründen der Volumenreduzierung und vor allem aus Gründen eines verbesserten Eigenschaftsprofils. Durch die erhöhte Konzentration an Superabsorbern kommt es nach erfolgter Flüssigkeitsaufnahme zu verstärktem Kontakt der gequollenen Absorberteilchen untereinander. Durch die im Stand der Technik beschriebenen Methoden der Oberflächennachvernetzung kann das sogenannte Gelblocking unterdrückt werden, bei dem nur die Oberflächen der Absorberteilchen anquellen und die Flüssigkeit nicht in die inneren Bereiche vordringt und miteinander verklebte, angequollene Absorberteilchen eine Sperrschicht für nachfolgende Flüssigkeit aufbauen.

Die Superabsorber werden deshalb nach dem Zerkleinern, Trocknen, Mahlen und Klassieren an der Oberfläche nachvernetzt.

Derartige Verfahren der Oberflächennachvernetzung sind beispielsweise in den Patentschriften DE 40 20 780 C1 und US 4,043,952 beschrieben. In der DE 40 20 780 C1 werden die Polymere an der Teilchenoberfläche durch niedermolekulare organische Verbindungen vernetzt. Damit wird nicht nur die Absorptionsfähigkeit unter Druck erhöht, sondern auch das als Gelblocking bekannte Verhalten der Absorber unterdrückt. Der US 4,043,952 ist eine Behandlung der Oberfläche der Absorberpartikel mit mindestens zweiwertigen Metallionen (Sp. 8, Zeile 51) in organischen Lösemitteln zur Verbesserung der Dispergierbarkeit in wässrigem Medium und zur schnelleren Aufnahme der Flüssigkeit zu entnehmen. Aus der EP 233 067 B1 bzw. der US 4,558,091 ist die Oberflächennachvernetzung von Superabsorbern auf Basis von Polyacrylsäure bzw. hydrolysiertem Stärke/Acrylnitril-Pfropfpolymer mit Aluminiumverbindungen in Kombination mit Polyhydroxyalkholen zur Verbesserung der Absorptionseigenschaften bekannt.

Während und nach der Oberflächennachvernetzung werden die Polymerpulver durch Misch- und Transportvorgänge in ihrem Komspektrum durch Bildung von feinteiligem Abrieb verändert, so dass eine erneute Absiebung der Feinanteile vonnöten ist, um den vorherigen Zustand wieder herzustellen. Dadurch entstehen zusätzliche Produktionskosten und Materialverluste, da die Feinanteile nur noch begrenzt oder gar nicht mehr einsetzbar sind. Zusätzlich zur Bildung des Abriebs erfolgt aber auch eine Verschlechterung der Absorptionseigenschaften, die zuvor durch die Oberflächennachvernetzung verbessert wurden, d.h. insbesondere auch die Fähigkeit des gequollenen Absorbergels weitere Flüssigkeit zu transportieren (Gelpermeabilität) wird verschlechtert. Dieses Problem stellt sich nicht nur bei der Herstellung der Superabsorberpulver, sondern letztlich auch bei deren Weiterverarbeitung in der Produktion zur Herstellung von Hygieneprodukten. Dort ist es häufig zu beobachten, dass die Absorptionseigenschaften der Superabsorber während der Förderung durch Abrieb verschlechtert werden und unerwünschtes Stauben auftritt.

Im Stand der Technik EP 691 995 A1 werden beispielsweise Maßnahmen zur Reduzierung des Staubanteiles durch Zusatz von Polyglykolen beschrieben, die allerdings nur das Stauben verhindern aber nicht die Probleme der verschlechterten Absorptionseigenschaften ausräumen. In der US 5,002,986 wird ein Verfahren zur Verbesserung der Absorptionsgeschwindigkeit von Superabsorbern, die nur aus Feinanteilen bestehen, beschrieben, die in Intensivmischern in Anwesenheit von ionischen Vernetzern zu größeren Teilchen agglomeriert werden. In der DE 196 46 484 A1 werden Superabsorber aus einer Kombination von speziellen Vernetzern und Monomeren beschrieben, die unter mechanischer Belastung einen geringeren Eigenschaftsabfall erleiden. Eine weitgehende Unterdrückung des Eigenschaftsverlustes ist aber auch dadurch nicht zu erreichen.

Aus dem Stand der Technik ist kein superabsorbierendes Polymer bekannt, dessen Eigenschaften durch die mechanische Beanspruchung während der Förderung bei Produktion und Windelfertigung nicht verschlechtert werden. Aus dem Stand der Technik ist außerdem kein Verfahren bekannt, das die Probleme der Schädigung der Eigenschaften der Superabsorberpulver durch die mechanische Beanspruchung während der Oberflächenmodifizierung und anschließender Förderung bei Produktion und Windelfertigung löst. Allenfalls wird eine Absiebung von Feinanteilen auf Kosten der Produktausbeute durchgeführt.

Aufgabe der vorliegenden Erfindung ist es deshalb Polymerisate zur Verfügung zu stellen, deren Eigenschaften sich während der Windelherstellung nur unwesentlich verschlechtern, die nicht oder wenig stauben und die eine geringere Tendenz zum Verklumpen in Umgebungen mit hoher Luftfeuchtigkeit aufweisen als Produkte des Standes der Technik. Aufgabe der vorliegenden Erfindung ist es außerdem ein Verfahren zur Herstellung von oben genannten Polymerisaten zur Verfügung zu stellen, durch das das Absieben der Feinanteile nach der Oberflächennachvernetzung weitgehend vermieden wird, ohne daß sich die Eigenschaften des Polymerisates wesentlich verschlechtern. Eine weitere Aufgabe der vorliegenden Erfindung ist es eine Substanz zur Verfügung zu stellen, durch die die Gelpermeabilität von durch Abriebvorgänge geschädigten, Feinanteile enthaltenden oberflächenvernetzten Superabsorberpulvern wiederhergestellt wird.

Die Aufgabe wird erfindungsgemäß durch ein pulverförmiges, an der Oberfläche nachvernetztes, Wasser oder wäßrige Flüssigkeiten absorbierendes Polymerisat gelöst, das aus polymerisierten gegebenenfalls vorvernetzten, teilneutralisierten Carboxylgruppen enthaltenden Monomeren aufgebaut ist, wobei das pulverförmige Polymerisat nach der Nachvernetzung mit der vorzugsweise wässrigen Lösung wenigstens eines Salzes eines mindestens dreiwertigen Kations umgesetzt worden ist.

Die erfindungsgemäßen Polymerisate weisen gegenüber Superabsorbern gemäß dem Stand der Technik verbesserte Gelpermeabilitäten auf, die in etwa denen von mechanisch nicht belasteten Polymerisaten entsprechen. Das Retentionsverhalten ist bei dem erfindungsgemäßen Polymerisat nicht reduziert und die Quellfähigkeit unter Druck nicht bzw. nur gering abgeschwächt. Ferner hat es sich herausgestellt, dass die erfindungsgemäßen Polymerisate eine gute Fliessfähigkeit besitzen und eine Verbesserung im sogenannten Anticakingverhalten aufweisen, d.h. bei feuchter Umgebungsluft nur eine geringe Neigung zum Zusammenbacken zeigen und darüber hinaus ein reduziertes Stauben zeigen. Dadurch können die üblicherweise für das Entstauben und die Anticakingbehandlung vorgesehenen Behandlungsschritte reduziert werden oder ganz entfallen. Die erfindungsgemäßen pulverförmigen Polymerisate ermöglichen eine sichere Weiterverarbeitung z.B. zu Windeln ohne Stauben und Eigenschaftsverlust bei mechanischer oder pneumatischer Förderung.

Als Salzkomponente in der Lösung können erfindungsgemäß Chloride, Bromide, Sulfate, Carbonate, Nitrate, Phosphate sowie Salze organischer Säuren wie z. B. Acetate und Lactate und andere Salze von mindestens dreiwertigen Kationen eingesetzt werden. Beispiele für erfindungsgemäß einsetzbare Kationen sind Aluminium, sowie Eisen, Chrom, Mangan, Titan, Zirkonium und andere Übergangsmetalle sowie Doppelsalze zweier Kationen oder auch Mischungen mehrerer Salze. Bevorzugt werden Aluminiumsalze und Alaune und deren unterschiedliche Hydrate wie z. B. $AlCl_3$ x 6 $H_2O$, $NaAl(SO_4)_2$ x 12 $H_2O$, $KAl(SO_4)_2$ x 12 $H_2O$ oder $Al_2(SO_4)_3$ x 18 $H_2O$. Besonders bevorzugt werden $Al_2(SO_4)_3$ und seine Hydrate eingesetzt. Vorzugsweise werden die Salzkomponenten, berechnet auf das erfindungsgemäße Kation in Mengen von 0,001 -1,0 Gew.%, bevorzugt 0,002 - 0,5 Gew.%, und besonders bevorzugt 0,005 - 0,2 Gew.%, jeweils bezogen auf das Polymerisat eingesetzt. Die Zugabemenge wird bevorzugt so bemessen, dass die Absorption des Polymerisatpulvers unter Druck keine bzw. nur geringe Einbussen erleidet.

Die erfindungsgemäß zu verwendenden Salze von mindestens dreiwertigen Kationen werden bevorzugt in Form einer Lösung aufgebracht. Geeignete Lösemittel sind Wasser oder polare, mit Wasser mischbare organische Lösemittel wie beispielsweise Aceton, Methanol, Ethanol oder 2-Propanol bzw. deren Gemische, bevorzugt wird Wasser verwendet. Der Begriff wässrige Lösung im Sinne der Erfindung bedeutet in Bezug auf die Lösemittelkomponente, dass neben dem Wasser auch noch andere organische Lösemittel enthalten sein können. Die Konzentration der Salze (wasserfrei berechnet) in dem Lösemittel kann in weiten Grenzen schwanken und liegt meistens im Bereich von 1 bis 80 Gew.%, vorzugsweise in einem Bereich von 1 bis 60 Gew.% und ganz besonders bevorzugt in einem Bereich von 5 bis 35 Gew.%. Das bevorzugte Lösungsmittel für die Salzkomponente ist Wasser, das in einer Menge von 0,05 - 10 Gew.%, bevorzugt 0,1 - 5 Gew.% und besonders bevorzugt 0,1-3 Gew.%, bezogen auf das Polymerisat verwendet wird. Die Wassermenge wird im unteren Bereich bevorzugt so bemessen, dass genügend Flüssigkeit zur Verteilung der Salzlösung zur Verfügung steht. Im oberen Bereich der Wassermenge muss dahingehend optimiert werden, dass die Bildung von Agglomeraten, die bei der Verwendung größerer Wassermengen temporär auftreten kann, in Grenzen bleibt. Generell gilt, dass mit steigender Menge Salz von mindestens dreiwertigen Kationen auch steigende Mengen Wasser ohne temporäre Agglomeratbildung verwendet werden können.

Als wasserquellbare hydrophile Polymerisate kommen natürliche, teilsynthetische und vollsynthetische Substanzen in Betracht. Bevorzugt sind teilsynthetische und vollsynthetische Substanzen, insbesondere anionische Polymerisate auf Basis von (Meth)Acrylsäure, die in teilneutralisierter Form als Alkalisalze, insbesondere Natrium- und/ oder Kaliumsalze, vorliegen. Der Neutralisationsgrad der sauren Monomerkomponente kann schwanken, liegt aber vorzugsweise zwischen 25 und 85 Mol.%. Es kann sich um Homo- und Copolymerisate handeln, die allein aus Acrylsäure und/oder Methacrylsäure erhältlich sind, aus diesen Monomeren zusammen mit einem oder mehreren anderen Monomeren oder allein aus einem oder mehreren anderen Monomeren, aber beispielsweise auch um aufgepfropfte anionische Polymerisate, z.B. auf Basis von (Meth)Acrylsäure, in teilneutralisierter Form als Alkalisalz vorliegend, z. B. um Pfropfpolymerisate auf Polyvinylalkohol, Polysacchariden wie etwa Stärke oder Cellulose oder Derivaten hiervon oder auf Polyalkylenoxiden, wie Polyethylenoxiden oder Polypropylenoxiden.

Als Beispiele für Monomere, die neben (Meth)Acrylsäure bei der Herstellung der Polymerisate Verwendung finden können, seien Methyl-, Ethyl-, und - (Poly)Hydroxyalkylester der (Meth)Acrylsäure, (Meth)Acrylamid, Crotonsäure, Malein- und Fumarsäure, Itaconsäure, 2-Acrylamido-2-methylpropansulfonsäure, Vinylsulfonsäure und Vinylphosphonsäure und die Methyl-, Ethyl- und (Poly)Hydroxyalkylester und Amide dieser Säuren, amino- und ammoniumgruppenhaltige Ester und Amide aller genannten Säuren und wasserlösliche N-Vinylamide genannt, aber auch aus allen weiteren, bei der Herstellung von Superabsorberpolymerisaten üblichen Monomeren stammende Baueinheiten können im Polymerisat enthalten sein. Das Polymerisat ist bevorzugt vernetzt. Als Beispiele für geeignete, bei der Herstellung der Superabsorberpolymerisate einsetzbare, zwei oder mehr reaktive Gruppen enthaltende Vernetzersubstanzen, deren Bauelemente dann im Polymerisat enthalten sein können, seien Polyglycidylether, Methylenbis(meth)acrylamid, Bisacrylamidoessigsäure, Ester ungesättigter Säuren von Polyolen bzw. alkoxylierten Polyolen, z.B. Ethylenglykoldi(meth)acrylat oder Trimethylolpropantriacrylat oder Allylverbindungen, wie z.B. Allyl(meth)acrylat, Polyallylester, Tetraallyloxyethan, Triallylamin, Tetraallylethylendiamin oder Allylester der Phosphorsäure sowie Vinylphosphonsäurederivate genannt. Der Anteil an Vernetzern, die bereits bei der Herstellung der Superabsorberpolymerisate zugegeben sind, liegt bevorzugt bei 0,01 bis 20 Gew.%, besonders bevorzugt bei 0,1 bis 3 Gew.%, bezogen auf die eingesetzten Gesamtmonomeren.

Die Herstellung des Polymerisates erfolgt ansonsten nach an sich bekannten Methoden, wie sie z.B. in der DE 40 20 780 C1, die hiermit als Referenz eingeführt wird und somit als Teil der Offenbarung gilt, beschrieben sind, bevorzugt durch Polymerisation in wässriger Lösung nach dem Verfahren der sogenannten Gelpolymerisation.

Die Polymerpulver, sind durch Zerkleinerung, Trocknung und Mahlen der Polymerisatgele und folgender Oberflächennachvernetzung entstanden und können ein breites Kornspektrum aufweisen. Für eine solche Oberflächennachvernetzung geeignete Substanzen sind z.B. Verbindungen, die zwei oder mehr Gruppen enthalten, die mit den Carboxylgruppen des hydrophilen Polymeren kovalente Bindungen bilden können. Geeignete Verbindungen sind beispielsweise Di- und Polyole, Di- und Polyglycidylverbindungen, wie Phosphonsäurediglycidylester, Alkylencarbonate, wie Ethylencarbonat, Alkoxysilylverbindungen, Polyaziridine, Polyamine oder Polyamidoamine, wobei die genannten Verbindungen auch in Mischungen untereinander verwendet werden können.

Das Oberflächennachvernetzungsmittel wird in einer Menge von 0,01 bis 30 Gewichtsprozent, bevorzugt 0,1-10 Gewichtsprozent, bezogen auf das nachzuvernetzende Polymer eingesetzt.

Vor der Oberflächennachvernetzung wird das Polymere vorzugsweise getrocknet, gemahlen und auf die für die jeweils anwendungstechnisch günstige Kornfraktion abgesiebt und dann der Oberflächennachvemetzungsreaktion zugeführt. In manchen Fällen hat es sich jedoch auch bewährt, die Oberflächennachvernetzer bereits vor der Trocknung des Polymergels bzw. vor der Zerkleinerung des teilweise oder überwiegend getrockneten Polymers zuzufügen. Eine erfindungsgemäß durchzuführende Oberflächennachvernetzung wird z.B. in der US 4 666 983 und der DE 40 20 780 beschrieben. Diese Schriften werden hiermit als Referenz eingeführt und gelten somit als Teil der Offenbarung. Der Zusatz der Oberflächennachvernetzer erfolgt häufig vorteilhafterweise auch in Form einer Lösung in Wasser, organischen Lösemitteln oder deren Mischungen, insbesondere dann, wenn geringe Mengen an Oberflächennachvernetzungsmittel angewandt werden. Geeignete Mischaggregate zum Aufbringen des Oberflächennachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer, sowie kontinuierlich arbeitende senkrechte Mischer in denen das Pulver mittels rotierender Messer in schneller Frequenz gemischt wird (Schugi-Mischer). Nachdem der Oberflächennachvernetzer mit dem vorvernetzten Polymer vermischt worden ist, wird zur Durchführung der Oberflächennachvernetzungsreaktion auf Temperaturen von 60 bis 250 °C, bevorzugt auf 135 bis 200°C und besonders bevorzugt auf 150 bis 185°C erhitzt. Die Zeitdauer der Nacherhitzung ist durch den Punkt begrenzt, bei dem das gewünschte Eigenschaftsprofil des Polymerisates infolge von Hitzeschädigung wieder zerstört wird.

Nach der Oberflächennachvernetzung wird das pulverförmige Polymerisat erfindungsgemäß mit der Lösung mindestens eines Salzes eines mindestens dreiwertigen Kations umgesetzt. Der Feuchtegehalt des Polymerisatpulvers vor der Umsetzung kann schwanken, typischerweise beträgt er weniger als 10 Gew.%, bevorzugt weniger als 8 Gew.% und besonders bevorzugt weniger als 5 Gew.%.

Das Polymerisatpulver kann vor der Umsetzung Feinanteile aufweisen. Dieser Feinanteil kann beim Trocknen, Mahlen und/oder Nachvernetzen entstanden sein oder dem Polymerisatpulver zugesetzt werden, so daß das erfindungsgemäße Polymerisat auch recyclierte Feinanteile enthalten kann. Vorzugsweise beträgt der Feinstaubanteil mit einem mittleren Korndurchmesser kleiner 150 µm bis zu 15 Gew.%, bevorzugt bis zu 10 Gew.% besonders bevorzugt bis zu 8 gew.% und ganz besonders bevorzugt bis zu 5 Gew.%. Für den Einsatz der Polymerpulver in der Hygieneindustrie hat sich eine obere Korngrenze von 1000 µm, bevorzugt von 850 µm bewährt.

Das Pulver und die Lösung wenigstens eines Salzes des mindestens dreiwertigen Kations werden vorzugsweise innig und möglichst homogen miteinander vermischt und dabei umgesetzt.

Die erfindungsgemäßen, pulverförmigen wasserquellbaren hydrophilen Polymerisate können für alle Zwecke verwendet werden, für die solche Superabsorber üblicherweise eingesetzt werden, insbesondere also zur Absorption von Wasser und wässrigen Lösungen. Bevorzugt finden sie Verwendung bei der Absorption von Körperflüssigkeiten, insbesondere von Blut und Urin. Hierzu werden sie insbesondere in absorbierende Einweg-Hygieneartikel, z.B. in Windeln, Tampons oder Damenbinden oder für andere medizinische Zwecke, eingearbeitet. Andere Verwendungszwecke sind z.B. die als wasserspeichernde Bodenverbesserungsmittel, bei der pflauzenaufzucht, insbesondere für Düngemittel, oder als Feuchtigkeitsbindemittel bei der Kabelummantelung oder als Trägermaterial für Wirkstoffe und deren kontrollierte Freisetzung sowie in Verpackungsmaterialien und in geschäumten und nicht geschänmten Flächengebilden.

Ein weiterer Gegenstand der vorliegenden Erfindung ist ein Verfahren zur Herstellung der erfindungsgemäßen Polymeren, bei dem dem pulverförmigen Polymerisat nach der Nachvernetzung eine Lösung wenigstens eines Salzes eines mindestens dreiwertigen Kations zugesetzt wird und das pulverförmige Polymerisat und die Lösung vorzugsweise homogen durchmischt werden.

Mit dem erfindungsgemäßen Verfahren ist es möglich, die Gelpermeabilität von Polymerisatpulvern mit hoher Abriebschädigung wieder aufzubauen. Die Stufe der Absiebung der Feinanteile nach der Nachvernetzung wird eingespart. Die Verfahrensprodukte weisen gegenüber Superabsorbern gemäß dem Stand der Technik verbesserte Gelpermeabilitäten auf, die in etwa denen von mechanisch nicht belasteten Polymerisaten entsprechen. Das Retentionsverhalten wird durch das erfinderische Verfahren nicht negativ beeinflusst und die Quellfähigkeit unter Druck nicht bzw. nur gering abgeschwächt. Ferner hat es sich herausgestellt, dass die pulverigen Verfahrensprodukte eine gute Fliessfähigkeit besitzen, eine Verbesserung im sogenannten Anticakingverhalten aufweisen, d.h. bei feuchter Umgebungsluft nur eine geringe Neigung zum Zusammenbacken und darüber hinaus ein reduziertes Stauben zeigen. Dadurch können die üblicherweise für das Entstauben und die Anticakingbehandlung vorgesehenen Behandlungsschritte reduziert werden oder ganz entfallen. Die erfindungsgemäßen Polymerpulver ermöglichen eine sichere Weiterverarbeitung ohne Stauben und Eigenschaftsverlust bei mechanischer oder pneumatischer Förderung. Ein weiterer Vorteil des erfindungsgemäßen Verfahrens ist die Tatsache, dass der über die Salzlösung eingebrachte Lösemittelanteil, insbesondere wenn er nur aus Wasser besteht, nicht abdestilliert werden muss, so dass das Polymerpulver ohne weitere Aufarbeitung einsatzfähig ist.

Erfindungsgemäß wird dem Polymerisatpulver nach der Nachvernetzung eine Lösung wenigstens eines Salzes eines mindestens dreiwertigen Kations zugesetzt. Während oder nach dem Zusatz der Lösung werden das pulverförmige Polymerisat und die Lösung vorzugsweise homogen durchmischt.

Der Feuchtegehalt des Polymerpulvers nach der Nachvernetzung und vor dem Zusatz der Lösung kann schwanken. Typischerweise beträgt er weniger als 10 Gew.%, bevorzugt weniger als 8 Gew.% und besonders bevorzugt weniger als 5 Gew.%.

Das pulverförmige Polymerisat kann vor dem Zusatz der Lösung Feinstaubanteile aufweisen, die entweder in der Nachvernetzung entstanden sind oder dem Polymerpulver zugesetzt werden, so daß sich das erfindungsgemäße Verfahren auch zum Recyclieren von Feinanteilen eignet. Vorzugsweise beträgt die Kornfraktion <150 µm bis zu 15 Gew.%, besonders bevorzugt bis zu 10 Gew.% und ganz besonders bevorzugt bis zu 5 Gew.%. Für den Einsatz der Polymerpulver in der Hygieneindustrie hat sich eine obere Korngrenze von 1000 µm, bevorzugt von 850 µm bewährt.

Erfindungsgemäß muß das Pulver innig und möglichst homogen mit der Lösung des Salzes des mindestens dreiwertigen Kations vermischt werden. Das Mischen kann in einer kontinuierlichen oder diskontinuierlichen Arbeitsweise erfolgen in jeder zur Mischung von pulverförmigen Produkten mit flüssigen Zusätzen geeigneten Apparatur. Vorzugsweise erfolgt die Durchmischung mit einem Rührwerk, das vorzugsweise bei einer Drehzahl von 700 - 1000 UpM betrieben wird. Dadurch wird insbesondere eine erneute Schädigung der Verfahrensprodukte vermieden. Die sonst für die Oberflächenbehandlung von superabsorbierenden Polymerpulvern im Stand der Technik (DE 41 31 045 C1) beschriebenen Intensivmischer mit hohem Energieeintrag von z.B. 1000 bis 5000 $Wh/m^3$ sind für das erfindungsgemäße Verfahren vorzugsweise nicht anzuwenden.

Die Mischzeiten liegen für gewöhnlich zwischen 1 und 120 Minuten, bevorzugt aber unter 1 Stunde. Temporäre Agglomerate, wie sie durch den Eintrag größerer Wassermengen entstehen können, werden durch die leichte Mischbewegung des Mischers wieder abgebaut, können aber eine Verlängerung der Mischzeit verursachen.

Der Zusatz der Lösung zu den pulverförmigen Polymerisaten findet vorzugsweise im Temperaturbereich von 0° C bis 100° C, besonders bevorzugt im Bereich von 10° C bis 80° C, ganz besonders bevorzugt im Bereich von 20° C bis 50° C statt.

Ein weiterer Gegenstand der vorliegenden Erfindung sind die Polymerisate, die aus dem erfindungsgemäßen Verfahren resultieren.

Die erfindungsgemäßen, pulverförmigen wasserquellbaren hydrophilen Polymerisate können für alle Zwecke verwendet werden, für die solche Superabsorber üblicherweise eingesetzt werden, insbesondere also zur Absorption von Wasser und wässrigen Lösungen. Bevorzugt finden sie Verwendung bei der Absorption von Körperflüssigkeiten, insbesondere von Blut und Urin. Hierzu werden sie insbesondere in absorbierende Einweg-Hygieneartikel, z.B. in Windeln, Tampons oder Damenbinden oder für andere medizinische Zwecke, eingearbeitet. Andere Verwendungszwecke sind z.B. die als wasserspeichernde Bodenverbesserungsmittel oder als Feuchtigkeitsbindemittel bei der Kabelummantelung oder als Trägermaterial für Wirkstoffe und deren kontrollierte Freisetzung.

Ein weiterer Gegenstand der vorliegenden Erfindung ist die Verwendung einer Lösung wenigstens eines Salzes eines mindestens dreiwertigen Kations zur Wiederherstellung der Gelpermeabilität von durch mechanische Einwirkung geschädigten pulverförmigen Wasser oder wässrige Flüssigkeiten absorbierenden Polymerisaten, aufgebaut aus polymerisierten gegebenenfalls vorvernetzten, teilneutralisierten Carboxylgruppen enthaltenden Monomeren, bei der dem pulverförmigen Polymerisat nach der Nachvernetzung die Lösung des Salzes zugesetzt wird und das pulverförmige Polymerisat und die Lösung durchmischt werden.

Mit der erfindungsgemäßen Verwendung ist es möglich, die Gelpermeabilität von Polymerpulvern mit hoher Abriebschädigung und hohen Feinanteilen wieder aufzubauen. Die Stufe der Absiebung der Feinanteile nach der Nachvernetzung wird eingespart. Die Verfahrensprodukte weisen gegenüber Superabsorbern gemäß dem Stand der Technik verbesserte Gelpermeabilitäten auf, die in etwa denen vor der mechanischen Belastung entsprechen. Das Retentionsverhalten wird durch die erfinderische Behandlung nicht negativ beeinflusst und die Quellfähigkeit unter Druck nicht bzw. nur gering abgeschwächt. Ferner hat es sich herausgestellt, dass die pulverigen Verfahrensprodukte eine gute Fliessfähigkeit besitzen, eine Verbesserung im sogenannten Anticakingverhalten aufweisen, d.h. bei feuchter Umgebungsluft nur eine geringe Neigung zum Zusammenbacken zeigen und darüber hinaus ein reduziertes Stauben zeigen. Dadurch können die üblicherweise für das Entstauben und die Anticakingbehandlung vorgesehenen Behandlungsschritte reduziert werden oder ganz entfallen. Die erfindungsgemäßen Polymerpulver ermöglichen eine sichere Weiterverarbeitung z.B. zu Windeln ohne Stauben und Eigenschaftsverlust bei mechanischer oder pneumatischer Förderung. Ein weiterer Vorteil des erfindungsgemäßen Verfahrens ist die Tatsache, dass der über die Salzlösung eingebrachte Lösemittelanteil, insbesondere wenn er nur aus Wasser besteht, nicht abdestilliert werden muss, so dass das Polymerpulver ohne weitere Aufarbeitung einsatzfähig ist.

Als Salzkomponente in der Lösung können erfindungsgemäß Chloride, Bromide, Sulfate, Carbonate, Nitrate, Phosphate sowie Salze organischer Säuren wie z. B. Acetate und Lactate und andere Salze von mindestens dreiwertigen Kationen eingesetzt werden. Beispiele für erfindungsgemäß einsetzbare Kationen sind Aluminium, sowie Eisen, Chrom, Mangan, Titan, Zirkonium und andere Übergangsmetalle sowie Doppelsalze zweier Kationen oder auch Mischungen mehrerer Salze. Bevorzugt werden Aluminiumsalze und Alaune und deren unterschiedliche Hydrate wie z. B. $AlCl_3$ x 6 $H_2O$, $NaAl(SO_4)_2$ x 12 $H_2O$, $KAl(SO_4)_2$ x 12 $H_2O$ oder $Al_2(SO_4)_3$ x 18 $H_2O$. Besonders bevorzugt werden $Al_2(SO_4)_3$ und seine Hydrate eingesetzt. Vorzugsweise werden die Salzkomponenten, berechnet auf das erfindungsgemäße Kation in Mengen von 0,001 -1,0 Gew.%, bevorzugt 0,002 - 0,5 Gew.%, und besonders bevorzugt 0,005 - 0,2 Gew.%, jeweils bezogen auf das Polymerisat eingesetzt. Die Zugabemenge wird bevorzugt so bemessen, dass die Absorption des Polymerisatpulvers unter Druck keine bzw. nur geringe Einbussen erleidet.

Die erfindungsgemäß zu verwendenden Salze von mindestens dreiwertigen Kationen werden bevorzugt in Form einer Lösung aufgebracht. Geeignete Lösemittel sind Wasser oder polare, mit Wasser mischbare organische Lösemittel wie beispielsweise Aceton, Methanol, Ethanol oder 2-Propanol bzw. deren Gemische, bevorzugt wird Wasser verwendet. Der Begriff wässrige Lösung im Sinne der Erfindung bedeutet in Bezug auf die Lösemittelkomponente, dass neben dem Wasser auch noch andere organische Lösemittel enthalten sein können. Die Konzentration der Salze (wasserfrei berechnet) in dem Lösemittel kann in weiten Grenzen schwanken und liegt meistens im Bereich von 1 bis 80 Gew.%, vorzugsweise in einem Bereich von 1 bis 60 Gew.% und ganz besonders bevorzugt in einem Bereich von 5 bis 35 Gew.%. Das bevorzugte Lösungsmittel für die Salzkomponente ist Wasser, das in einer Menge von 0,05 - 10 Gew.%, bevorzugt 0,1 - 5 Gew.% und besonders bevorzugt 0,1-3 Gew.%, bezogen auf das Polymerisat verwendet wird. Die Wassermenge wird im unteren Bereich bevorzugt so bemessen, dass genügend Flüssigkeit zur Verteilung der Salzlösung zur Verfügung steht. Im oberen Bereich der Wassermenge muss dahingehend optimiert werden, dass die Bildung von Agglomeraten, die bei der Verwendung größerer Wassermengen temporär auftreten kann, in Grenzen bleibt. Generell gilt, dass mit steigender Menge Salz von mindestens dreiwertigen Kationen auch steigende Mengen Wasser ohne temporäre Agglomeratbildung verwendet werden können.

Als wasserquellbare hydrophile Polymerisate kommen natürliche, teilsynthetische und vollsynthetische Substanzen in Betracht. Bevorzugt sind teilsynthetische und vollsynthetische Substanzen, insbesondere anionische Polymerisate auf Basis (Meth)Acrylsäure, die in teilneutralisierter Form als Alkalisalze, insbesondere Natrium- und/oder Kaliumsalze, vorliegen. Der Neutralisationsgrad der sauren Monomerkomponenten kann schwanken, liegt aber vorzugsweise zwischen 25 und 85 Mol.%. Es kann sich um Homo- und Copolymerisate handeln, die allein aus Acrylsäure und/oder Methacrylsäure erhältlich sind, aus diesen Monomeren zusammen mit einem oder mehreren anderen Monomeren oder allein aus einem oder mehreren anderen Monomeren, aber beispielsweise auch um aufgepfropfte anionische Polymerisate, z.B. auf Basis (Meth)Acrylsäure, in teilneutralisierter Form als Alkalisalz vorliegend, z.B. um Pfropfpolymerisate auf Polyvinylalkohol, Polysacchariden wie etwa Stärke oder Cellulose oder Derivaten hiervon oder auf Polyalkylenoxiden, wie Polyethylenoxiden oder Polypropylenoxiden.

Als Beispiele für Monomere, die neben (Meth)Acrylsäure bei der Herstellung der Polymerisate Verwendung finden können, seien Methyl-, Ethyl-, und - (Poly)Hydroxyalkylester der (Meth)Acrylsäure, (Meth)Acrylamid, Crotonsäure, Malein- und Fumarsäure, Itaconsäure, 2-Acrylamido-2-methylpropansulfonsäure, Vinylsulfonsäure und Vinylphosphonsäure und die Methyl-, Ethyl- und (Poly)Hydroxyalkylester und Amide dieser Säuren, amino- und ammoniumgruppenhaltige Ester und Amide aller genannten Säuren und wasserlösliche N-Vinylamide genannt, aber auch aus allen weiteren, bei der Herstellung von Superabsorberpolymerisaten üblichen Monomeren stammende Baueinheiten können im Polymerisat enthalten sein. Das Polymerisat ist bevorzugt vernetzt. Als Beispiele für geeignete, bei der Herstellung der Superabsorberpolymerisate einsetzbare, zwei oder mehr reaktive Gruppen enthaltende Vernetzersubstanzen, deren Bauelemente dann im Polymerisat enthalten sein können, seien Polyglycidylether, Methylenbis(meth) acrylamid, Bisacrylamidoessigsäure, Ester ungesättigter Säuren von Polyolen bzw. alkoxylierten Polyolen, z.B. Ethylenglykoldi(meth)acrylat oder Trimethylolpropantriacrylat oder Allylverbindungen, wie z.B. Allyl(meth)acrylat, Polyallylester, Tetraallyloxyethan, Triallylamin, Tetraallylethylendiamin oder Allylester der Phosphorsäure sowie Vinylphosphonsäurederivate genannt. Der Anteil an Vernetzern, die bereits bei der Herstellung der Superabsorberpolymerisate zugegeben sind, liegt bevorzugt bei 0,01 bis 20 Gew.%, besonders bevorzugt bei 0,1 bis 3 Gew.%, bezogen auf die eingesetzten Gesamtmonomeren.

Die Herstellung des Polymerisates erfolgt ansonsten nach an sich bekannten Methoden, wie sie z.B. in der DE 40 20 780 C1, die hiermit als Referenz eingeführt wird und somit als Teil der Offenbarung gilt, beschrieben sind. Bevorzugt erfolgt die Herstellung durch Polymerisation in wässriger Lösung nach dem Verfahren der sogenannten Gelpolymerisation.

Die Polymerpulver, sind durch Zerkleinerung, Trocknung und Mahlung der Polymerisatgele und folgender Oberflächennachvernetzung entstanden und können ein breites Kornspektrum aufweisen. Für eine solche Oberflächennachvernetzung geeignete Substanzen sind z.B. Verbindungen, die zwei oder mehr Gruppen enthalten, die mit den Carboxylgruppen des hydrophilen Polymeren kovalente Bindungen bilden können. Geeignete Verbindungen sind beispielsweise Di- und Polyole, Di- und Polyglycidylverbindungen, wie Phosphonsäurediglycidylester, Alkylencarbonate, wie Ethylencarbonat, Alkoxysilylverbindungen, Polyaziridine, Polyamine oder Polyamidoamine, wobei die genannten Verbindungen auch in Mischungen untereinander verwendet werden können.

Vor der Oberflächennachvernetzung wird das Polymere vorzugsweise getrocknet, gemahlen und auf die für die jeweils anwendungstechnisch günstige Kornfraktion abgesiebt und dann der Oberflächennachvernetzungsreaktion zugeführt. In manchen Fällen hat es sich jedoch auch bewährt, die Oberflächennachvernetzer bereits vor der Trocknung des Polymergels bzw. vor der Zerkleinerung des teilweise oder überwiegend getrockneten Polymers zuzufügen. Eine erfindungsgemäß durchzuführende Oberflächennachvernetzung wird z.B. in der US 4 666 983 und der DE 40 20 780 beschrieben. Diese Schriften werden hiermit als Referenz eingeführt und gelten somit als Teil der Offenbarung. Der Zusatz der Oberflächennachvernetzer erfolgt häufig vorteilhafterweise auch in Form einer Lösung in Wasser, organischen Lösemitteln oder deren Mischungen, insbesondere dann, wenn geringe Mengen an Oberflächennachvernetzungsmittel angewandt werden. Geeignete Mischaggregate zum Aufbringen des Oberflächennachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer, sowie kontinuierlich arbeitende senkrechte Mischer in denen das Pulver mittels rotierender Messer in schneller Frequenz gemischt wird (Schugi-Mischer). Nachdem der Oberflächennachvernetzer mit dem vorvernetzten Polymer vermischt worden ist, wird zur Durchführung der Oberflächennachvernetzungsreaktion auf Temperaturen von 60 bis 250 °C, bevorzugt auf 135 bis 200°C und besonders bevorzugt auf 150 bis 185°C erhitzt. Die Zeitdauer der Nacherhitzung ist durch den Punkt begrenzt, bei dem das gewünschte Eigenschaftsprofil des Polymerisates infolge von Hitzeschädigung wieder zerstört wird.

Nach der Oberflächennachvernetzung wird dem pulverförmigen Polymerisat erfindungsgemäß die Lösung mindestens eines Salzes eines mindestens dreiwertigen Kations innig zugesetzt und innig vermischt. Der Feuchtegehalt des Polymerpulvers vor dem Zusatz der Lösung kann schwanken. Typischerweise beträgt er weniger als 10 Gew. %, bevorzugt weniger als 8 Gew.% und besonders bevorzugt weniger als 5 Gew.%.

Das Polymerisatpulver kann vor dem Zusatz Feinanteile aufweisen. Dieser Feinanteil kann beim Trocknen, Malen und/oder Nachvemetzen entstanden sein oder dem Polymerpulver zugesetzt werden, so daß das erfindungsgemäße Polymerisat auch recyclierte Feinanteile enthalten kann. Vorzugsweise beträgt der Feinstaubanteil mit einem mittleren Korndurchmesser kleiner 150 μm bis zu 15 Gew.%, bevorzugt bis zu 10 Gew.% besonders bevorzugt bis zu 8 Gew.-% und ganz besonders bevorzugt bis zu 5 Gew.% eingesetzt. Für den Einsatz der Polymerpulver in der Hygieneindustrie hat sich eine obere Korngrenze von 1000 μm, bevorzugt von 850 μm bewährt.

Erfindungsgemäß wird dem Polymerisatpulver nach der Nachvernetzung eine Lösung wenigstens eines Salzes eines mindestens dreiwertigen Kations zugesetzt. Während oder nach dem Zusatz der Lösung werden das pulverförmige Polymerisat und die Lösung vorzugsweise homogen durchmischt.

Der Feuchtegehalt des Polymerpulver nach der Nachvernetzung und vor dem Zusatz der Lösung kann schwanken. Typischerweise beträgt er weniger als 10 Gew.%, bevorzugt weniger als 8 Gew.% und besonders bevorzugt weniger als 5 Gew.%.

Das pulverförmige Polymerisat kann vor dem Zusatz der Lösung Feinstaubanteile aufweisen, die entweder in der Nachvernetzung entstanden sind oder dem Polymerpulver zugesetzt werden, so daß sich die erfindungsgemäße Verwendung auch zum Recyclieren von Feinstaubanteilen eignet. Vorzugsweise beträgt die Kornfraktion <150 μm bis zu 15 Gew.%, besonders bevorzugt bis zu 10 Gew.% und ganz besonders bevorzugt bis zu 5 Gew.%. Für den Einsatz der Polymerpulver in der Hygieneindustrie hat sich eine obere Korngrenze von 1000 μm, bevorzugt von 850 μm bewährt.

Erfindungsgemäß muß das Pulver innig und möglichst homogen mit der Lösung des Salzes des mindestens dreiwertigen Kations vermischt werden. Das Mischen kann in einer kontinuierlichen oder diskontinuierlichen Arbeitsweise erfolgen in jeder zur Mischung von pulverförmigen Produkten mit flüssigen Zusätzen geeigneten Apparatur. Vorzugsweise erfolgt die Durchmischung mit einem Rührwerk, das vorzugsweise bei einer Drehzahl von 700 - 1000 UpM betrieben wird. Dadurch wird insbesondere eine erneute Schädigung der Verfahrensprodukte vermieden. Die sonst für die Oberflächenbehandlung von superabsorbierenden Polymerpulvem im Stand der Technik (DE 41 31 045 C1) beschriebenen Intensivmischer mit hohem Energieeintrag von z. B. 1000 bis 5000 Wh/m$^3$ sind für das erfindungsgemäße Verfahren vorzugsweise nicht anzuwenden.

Die Mischzeiten liegen für gewöhnlich zwischen 1 und 120 Minuten, bevorzugt aber unter 1 Stunde. Temporäre Agglomerate, wie sie durch den Eintrag größerer Wassermengen entstehen können, werden durch die leichte Mischbewegung des Mischers wieder abgebaut, können aber eine Verlängerung der Mischzeit verursachen.

Der Zusatz der Lösung zu dem pulverförmigen Polymerisat findet im Temperaturbereich von 0° C bis 100° C, besonders bevorzugt im Bereich von 10° C bis 80° C, ganz besonders bevorzugt im Bereich von 20° C bis 50° C statt. Temperaturen über 100°C bzw. nachfolgende Temperaturbehandlungen sind unzweckmäßig, da sie entweder keine Verbesserung der Eigenschaften bewirken oder sogar zu einer Verschlechterung der Eigenschaften führen können.

Die erfindungsgemäßen Polymerisate sowie die nach dem erfindungsgemäßen Verfahren oder der erfindungsgemäßen Verwendung anfallenden Superabsorber werden vorzugsweise in absorbierenden Hygieneprodukten wie etwa Babywindeln, Inkontinenzprodukten und Damenbinden eingesetzt.

Absorbierende Hygieneprodukte besitzen in der Regel einen allgemeinen Aufbau aus einer körperzugewandten, flüssigkeitsdurchlässigen Abdeckung, einer flüssigkeitsabsorbierenden Sauglage sowie einer im wesentlichen flüssigkeitsundurchlässigen, körperabgewandten Außenschicht. Optional finden auch weitere Konstruktionen zur schnellen Aufnahme und Verteilung von Körperflüssigkeit im Saugkern Anwendung. Diese Konstruktionen werden häufig, aber nicht zwingend zwischen der körperzugewandten, flüssigkeitsdurchlässigen Abdeckung und der flüssigkeitsabsorbierenden Sauglage eingesetzt.

Die flüssigkeitsdurchlässige Abdeckung besteht in der Regel aus einem nichtgewebten, faserigen Vlies oder einer anderen porösen Konstruktion.

Als Materialien für diese Abdeckung kommen z. B. synthetische Polymere wie etwa Polyvinylchlorid oder —fluorid, Polytetrafluorethylen (PTFE), Polyvinylalkohole und - Derivate, Polyacrylate, Polyamide, Polyester, Polyurethane, Polystyrol, Polysiloxane oder Polyolefine (z.B. Polyethylen (PE) oder Polypropylen (PP)) sowie natürliche Fasermaterialien sowie beliebige Kombinationen aus den vorgenannten Materialien im Sinne von Mischmaterialien oder Verbundmaterialien oder Copolymerisaten in Frage.

Die flüssigkeitsdurchlässige Abdeckung hat hydrophilen Charakter. Sie kann zudem aus einer Kombination von hydrophilen und hydrophoben Bestandteilen bestehen. Bevorzugt ist in der Regel eine hydrophile Ausrüstung der flüssigkeitsdurchlässigen Abdeckung, um schnelle Einsickerzeiten von Körperflüssigkeit in die flüssigkeitsabsorbierende Sauglage zu ermöglichen, jedoch werden auch partiell hydrophobierte Abdeckungen verwendet.

Flüssigkeitsabsorbierende Sauglage

Die flüssigkeitsabsorbierende Sauglage enthält die superabsorbierenden Pulver bzw. Granulate und weitere Komponenten aus beispielsweise faserigen Materialien, schaumförmigen Materialien, filmbildenden Materialien oder porösen Materialien sowie Kombinationen von zwei oder mehreren dieser Materialien. Jedes dieser Materialien kann entweder natürlichen oder synthetischen Ursprungs sein oder durch chemische oder physikalische Modifikation von natürlichen Materialien hergestellt worden sein. Die Materialien können hydrophil oder hydrophob sein, wobei hydrophile Materialien bevorzugt sind. Dies gilt insbesondere für solche Zusammensetzungen, die ausgeschiedene Körperflüssigkeiten effizient aufnehmen und in Richtung zu weiter von der Eintrittsstelle der Körperflüssigkeit entfernte Regionen des absorbierenden Kerns transportieren sollen.

Als hydrophile Fasermaterialien sind geeignet z.B. Cellulosefasern, modifizierte Cellulosefasern (z.B. versteifte Cellulosefasern), Polyesterfasern (z.B. Dacron), hydrophiles Nylon oder aber auch hydrophilisierte hydrophobe Fasern, wie z. B. mit Tensiden hydrophilisierte Polyolefine (PE, PP), Polyester, Polyacrylate, Polyamide, Polystyrol, Polyurethane und andere.

Bevorzugt werden Cellulosefasern und modifizierte Cellulosefasern eingesetzt.

Kombinationen von Cellulosefasern und/oder modifizierten Cellulosefasern mit synthetischen Fasern wie z. B. PE/PP Verbundmaterialien, sogenannte Bikomponentenfasern, wie sie z.B. zur Thermobondierung von Airlaidmaterialien verwendet werden oder anderen Materialien sind ebenfalls gebräuchlich.

Die Fasermaterialien können in verschiedenen Anwendungsformen vorliegen, z.B. als lose aus einem Luftstrom oder aus wässriger Phase abgeschiedene oder abgelegte Cellulosefasern, als nichtgewebtes Vlies oder als Tissue. Kombinationen verschiedener Anwendungsformen sind möglich.

Optional können neben den erfindungsgemäßen Superabsorbern weitere pulverförmige Substanzen eingesetzt werden, wie z.B. geruchsbindende Substanzen wie Cyclodextrine, Zeolithe, anorganische oder organische Salze und ähnliche Materialien.

Als poröse Materialien und schaumförmige Materialien können z.B. Polymerschäume eingesetzt werden, wie sie in den Schriften DE 44 18 319 A1 und DE 195 05 709 A1 beschrieben sind, die hiermit als Referenz eingeführt werden und somit als Teil der Offenbarung gelten.

Zur mechanischen Stabilisierung der flüssigkeitsabsorbierenden Sauglage können thermoplastische Fasern (z.B. Bikomponentenfasern aus Polyolefinen, Polyolefingranulate, Latexdispersionen oder Heisskleber) verwendet werden. Optional werden eine oder mehrere Lagen Tissue zur Stabilisierung verwendet.

Die flüssigkeitsabsorbierende Sauglage kann einlagig sein oder aus mehreren Schichten bestehen. Bevorzugt werden Konstruktionen verwendet, die aus hydrophilen Fasern, bevorzugt Cellulosefasern, optional einer Konstruktion zur schnellen Aufnahme und Verteilung von Körperflüssigkeit wie zum Beispiel chemisch versteifte (modifizierte) Cellulosefasern oder Highloftvliese aus hydrophilen oder hydrophilisierten Fasern sowie superabsorbierenden Polymeren bestehen.

Das erfindungsgemäße superabsorbierende Polymer kann dabei homogen in den Cellulosefasern oder den versteiften Cellulosefasern verteilt sein, es kann lagig zwischen den Cellulosefasern oder den versteiften Cellulosefasern eingebracht sein, oder die Konzentration des superabsorbierenden Polymers kann innerhalb der Cellulosefasern oder versteiften Cellulosefasern einen Gradienten aufweisen. Das Verhältnis der Gesamtmenge an superabsorbierendem Polymer und der Gesamtmenge an Cellulosefasern oder den versteiften Cellulosefasern im absorbierenden Saugkern kann zwischen 0 zu 100 und 80 zu 20 % variieren, wobei in einer Ausführungsform lokal, z. B. bei Gradienteneintrag oder schichtweisem Eintrag Konzentrationen von bis zu 100 % Superabsorber erreicht werden können. Derartige Konstruktionen mit Bereichen hoher Konzentrationen von absorbierendem Polymer, wobei der Anteil von Superabsorber in bestimmten Bereichen zwischen 60 und 100 %, am stärksten bevorzugt zwischen 90 % und 100 % liegt, sind beispielsweise auch in der Patentschrift US 5,669,894 beschrieben, die hiermit als Referenz eingeführt wird und somit als Teil der Offenbarung gilt.

Optional können auch mehrere verschiedene Superabsorber, die sich zum Beispiel in der Sauggeschwindigkeit, der Permeabilität, der Speicherkapazität, der Absorption gegen Druck, der Kornverteilung oder auch der chemischen Zusammensetzung unterscheiden, gleichzeitig eingesetzt werden. Die verschiedenen Superabsorber können miteinander vermischt in das Saugkissen eingebracht werden oder aber lokal differenziert im Absorbent Core plaziert werden. Eine solche differenzierte Plazierung kann in Richtung der Dicke des Saugkissens oder der Länge oder Breite des Saugkissens erfolgen.

In der flüssigkeitsabsorbierenden Sauglage befindet sich eine oder mehrere der oben beschriebenen, superabsorbierende Polymere enthaltenden Lagen aus Cellulosefasern oder versteiften Cellulosefasern. In einer bevorzugten Ausführungsform werden Konstruktionen aus Kombinationen von Lagen mit homogenem Superabsorbereintrag und zusätzlich schichtweiser Einbringung verwendet.

Optional werden diese erwähnten Strukturen auch durch weitere Lagen von reinen Cellulosefasern oder versteiften Cellulosefasern an der körperzugewandten Seite und / oder auch der körperabgewandten Seite ergänzt.

Die oben beschriebenen Konstruktionen können sich auch mehrfach wiederholen, wobei es sich um eine Aufeinanderschichtung zweier oder mehrerer gleicher Lagen oder aber auch um Aufeinanderschichtung zweier oder mehrerer unterschiedlicher Konstruktionen handeln kann. Dabei liegen die Unterschiede in wiederum rein konstruktiver Art oder aber im Typ des verwendeten Materials, wie z.B. die Verwendung von in den Eigenschaften differierender absorbierender Polymere oder aber verschiedener Zellstoffarten.

Optional sind das gesamte Saugkissen oder aber auch einzelne Lagen der flüssigkeitsabsorbierenden Sauglage durch Lagen von Tissue von anderen Komponenten getrennt oder stehen in direktem Kontakt mit anderen Lagen oder Komponenten.

Exemplarisch können zum Beispiel die Konstruktion zur schnellen Aufnahme und Verteilung von Körperflüssigkeit und die flüssigkeitsabsorbierende Sauglage durch Tissue voneinander getrennt sein oder aber in direktem Kontakt miteinander stehen. Sofern keine separate Konstruktion zur schnellen Aufnahme und Verteilung von Körperflüssigkeit zwischen der flüssigkeitsabsorbierenden Sauglage und der körperzugewandten flüssigkeitsdurchlässigen Abdeckung existiert, sondern der Effekt der Flüssigkeitsverteilung z.B durch die Verwendung einer speziellen körperzugewandten, flüssigkeitsdurchlässigen Abdeckung erreicht werden soll, kann die flüssigkeitsabsorbierende Sauglage ebenfalls optional von der körperzugewandten, flüssigkeitsdurchlässigen Abdeckung durch ein Tissue getrennt sein.

Statt Tissue kann optional auch nichtgewebtes Vlies in die flüssigkeitsabsorbierende Sauglage eingebracht werden. Beide Komponenten führen zu dem erwünschten Nebeneffekt der Stabilisierung und Festigung des Absorptionskerns im feuchten Zustand.

Herstellverfahren der flüssigkeitsabsorbierenden Sauglage

Faserhaltige, superabsorberhaltige, flüssigkeitsverteilende und -speichernde Schichten lassen lassen sich mit einer Vielzahl von Herstellverfahren generieren.

Neben den etablierten konventionellen Prozessen, wie Sie vom Fachmann allgemein unter Drumforming mit Hilfe von Formrädern, -taschen und Produktformen und entsprechend angepassten Dosiereinrichtungen für die Rohstoffe zusammengefaßt werden, sind moderne etablierte Verfahren wie der Airlaidprozess (z.B. EP 850 615, Sp. 4 Zeile 39 bis Sp. 5 Zeile 29, US 4.640.810) mit allen Formen der Dosierung, Ablage der Fasern und Verfestigung wie Hydrogenbonding (z.B. DE 197 50 890, Sp. 1 Zeile 45 bis Sp. 3 Zeile 50, Thermobonding, Latexbonding (z.B. EP 850 615, Sp. 8 Zeile 33 bis Sp. 9 Zeile 17 und Hybridbonding, der Wetlaid Prozeß (z.B. PCT WO 99/49905, Sp. 4 Zeile 14 bis Sp. 7 Zeile 16) , Carding-, Meltblown-, Spunblown-Prozesse sowie ähnliche Prozesse zur Herstellung von superabsorberhaltigen Non-Wovens (im Sinne der der Definition der EDANA, Brüssel) auch in Kombinationen dieser Verfahren mit- und untereinander übliche Methoden zur Herstellung von den o.g. Flüssigkeitsspeichern. Die oben genannten Schriften werden als Referenz eingeführt und gelten somit als Teil der Offenbarung.

Als weitere Verfahren kommen die Herstellung von Laminaten im weitesteten Sinne sowie von extrudierten und coextrudierten, naß- und trocken- sowie nachträglich verfestigten Strukturen in Frage.

Eine Kombinationen dieser Verfahren mit- und untereinander ist ebenfalls möglich.

Konstruktionen zur schnellen Aufnahme und Verteilung von Körperflüssigkeit

Eine Konstruktion zur schnellen Aufnahme und Verteilung von Körperflüssigkeit besteht zum Beispiel aus chemisch versteiften (modifizierten) Cellulosefasern oder Highloftvliesen aus hydrophilen oder hydrophilisierten Fasern oder einer Kombination von beidem.

Chemisch versteifte, modifizierte Cellulosefasern können zum Beispiel erzeugt werden aus Cellulosefasern, die durch Vernetzer wie z.B. $C_2$ - $C_8$ Dialdehyde, $C_2$ - $C_8$ Monoaldehyde mit einer zusätzlichen Säurefunktion oder $C_2$ - $C_9$ Polycarbonsäuren in einer chemischen Reaktion umgesetzt werden. Spezielle Beispiele sind: Glutaraldehyd, Glyoxal, Glyoxalsäure oder Zitronensäure. Ebenfalls bekannt sind kationisch modifizierte Stärke oder Polyamid-Epichlorhydrinharze (z. B. KYMENE 557H, Hercules Inc., Wilmington , Delaware). Durch die Vernetzung wird eine verdrehte, gekräuselte Struktur erreicht und stabilisiert, die sich vorteilhaft auf die Geschwindigkeit der Flüssigkeitaufnahme auswirkt.

Flächengewicht und Dichte von absorbierenden Artikeln

Die absorbierenden Hygieneprodukte können in ihrem Flächengewicht und Dicke und damit der Dichte stark variieren. Typischerweise liegen die Dichten der Bereiche der Absorptionskerne zwischen 0,08 und 0,25 $g/cm^3$. Die Flächengewichte liegen zwischen 10 und 1000 $g/m^2$, wobei bevorzugt Flächengewichte zwischen 100 und 600 $g/m^2$ realisiert werden (siehe auch US 5,669,894, die hiermit als Referenz eingeführt wird und somit als Teil der Offenbarung gilt). Die Dichte variiert in der Regel über die Länge des absorbierenden Kems. Dies tritt als Folge einer gezielten Dosierung der Cellulosefaser- oder versteiften Cellulosefasermenge oder der Menge des superabsorbierenden Polymers ein, da diese Komponenten in bevorzugten Ausführungsformen stärker in den Frontbereich des absorbierenden Einwegartikels eingebracht werden.

Diese gezielte Erhöhung des absorbierenden Materials in bestimmten Regionen des absorbierenden Kerns kann auf andere Weise auch z.B. durch Herstellung eines entsprechend großen, mittels eines Airlaid- oder Wetlaidverfahrens hergestellten Flächengebildes, bestehend aus hydrophilen Cellulosefasern, optional aus versteiften Cellulosefasern, optional aus synthetischen Fasern (z.B. Polyolefinen) sowie aus superabsorbierenden Polymeren und anschließender Rückfaltung oder Übereinanderlegen erreicht werden.

**Prüfmethoden**

**Prüfmethode 1**: Retention (TB)

Die Retention wird nach der Teebeutelmethode und als Mittelwert aus drei Messungen angegeben. Etwa 200 mg Polymerisat werden in einen Teebeutel eingeschweißt und für 30 Minuten in 0,9%ige NaCl-Lösung getaucht. Anschließend wird der Teebeutel in einer Schleuder (23 cm Durchmesser, 1.400 Upm) 3 Minuten geschleudert und gewogen. Einen Teebeutel ohne wasserabsorbierendes Polymerisat lässt man als Blindwert mitlaufen.

Retention = Auswaage-Blindwert /Einwaage [g/g]

**Prüfmethode 2:** Anticaking Test und Feuchtigkeitsaufnahme

Dieser Test soll zur Beurteilung des Caking-Verhaltens und zur Ermittlung der Feuchtigkeitsaufnahme dienen. Dazu werden 5 g Superabsorber in eine Schale eingewogen und gleichmäßig verteilt und über 3 Stunden bei 38 °C und 80 % rel. Luftfeuchte in einem Klimaschrank gelagert. Zur Ermittlung der Feuchtigkeitsaufnahme wird die Schale danach zurückgewogen. Das Caking-Verhalten bestimmt sich aus dem % Siebdurchgang der auf ein Sieb der Maschenweite 1,68 mm geschütteten Superabsorberprobe nach dreimaligem Anschlagen des Siebes. Superabsorber mit einem guten Anticaking-Verhalten bilden bei der Feuchtelagerung wenig Zusammenbackungen und passieren das Sieb fast vollständig.

**Prüfmethode 3:** Gelpermeabilität (SFC)

Die Prüfung wird nach einer in der WO 95/22356, die hiermit als Referenz eingeführt wird und somit als Teil der Offenbarung gilt, veröffentlichten Methode durchgeführt. In einen Zylinder mit Siebboden werden ca. 0,9 g Superabsorbermaterial eingewogen und sorgfältig auf der Siebfläche verteilt. Das Superabsorbermaterial lässt man in JAYCO synthetischen Urin 1 Stunde lang gegen einen Druck von 20 g/cm$^2$ quellen. Nach Erfassung der Quellhöhe des Superabsorbers lässt man bei konstantem hydrostatischem Druck 0,118 M NaCl-Lösung aus einem nivellierten Vorratsgefäß durch die gequollene Gelschicht laufen. Die gequollene Gelschicht ist während der Messung mit einem speziellen Siebzylinder abgedeckt, der eine gleichmäßige Verteilung der 0,118 M NaCl-Lösung oberhalb des Gels und konstante Bedingungen (Messtemperatur 20-25°C) während der Messung bezüglich der Gelbettbeschaffenheit gewährleistet. Der auf den gequollenen Superabsorber wirkende Druck ist weiterhin 20 g/cm$^2$. Mit Hilfe eines Computers und einer Waage wird die Flüssigkeitsmenge, die die Gelschicht als Funktion der Zeit passiert in Intervallen von 20 Sekunden innerhalb einer Zeitperiode von 10 Minuten erfasst. Die Fließrate g/s durch die gequollene Gelschicht wird mittels Regressionsanalyse mit Extrapolation der Steigung und Ermittlung des Mittelpunkts auf den Zeitpunkt t=0 der Fließmenge innerhalb der Minuten 2-10 ermittelt. Die Berechnung des SFC-Wertes (K) berechnet sich wie folgt:

$$K = \frac{F_s(t=0) \cdot L_o}{r \cdot A \cdot \Delta P} = \frac{F_s(t=0) \cdot L_0}{139506}$$

Wobei:

$F_s$ (t = 0)  die Fließrate in g/s
$L_0$  die Dicke der Gelschicht in cm
r  die Dichte der NaCl-Lösung (1,003 g/cm$^3$)
A  die Fläche der Oberseite der Gelschicht im Messzylinder (28,27 cm$^2$)
$\Delta P$  der hydrostatische Druck, der auf der Geschicht lastet (4920 dyne/cm$^2$)
und K  der SFC-Wert ist [cm$^3$ * s * g$^{-1}$]

**Prüfmethode 4:** Flüssigkeitsaufnahme unter Druck (AAP-Test)

Die Aufnahme unter Druck (Druckbelastung 50 g/cm$^2$) wird nach einer in der EP 0339461, Seite 7, beschriebenen Methode bestimmt. In einen Zylinder mit Siebboden werden ca. 0,9 g Superabsorber eingewogen. Die gleichmäßig aufgestreute Superabsorberlage wird mit einem Stempel belastet, der einen Druck von 50 g/cm$^2$ ausübt. Der zuvor gewogene Zylinder wird anschließend auf eine Glasfilterplatte gestellt, die sich in einer Schale mit 0,9%iger NaCl-Lösung befindet, deren Flüssigkeitniveau genau der Höhe der Filterplatte entspricht. Nachdem man die Zylindereinheit 1 Stunde lang 0,9%ige NaCl-Lösung saugen gelassen hat, wird diese zurückgewogen und der AAP wie folgt berechnet:

AAP = Auswaage (Zylindereinheit + vollgesogener Superabsorber)-Einwaage (Zylindereinheit + Superabsorber) / Einwaage Superabsorber

**Prüfmethode 5:** Fliessfähigkeit (FFC-Wert)

Die Fliessfähigkeit der superabsorbierenden Polymerpulver wird mit dem Ringschergerät RST-=1.01 der Firma Dr.-Ing. Dietmar Schulze Schüttgutmeßtechnik bestimmt. Der FFC-Wert gibt Auskunft über die Fliesseigenschaften eines Schüttgutes in einem Silo. Bei der Messung wird das Schüttgut in einer ringförmigen Scherzelle verschiedenen Belastungen ausgesetzt (Anscheren 500 000 Pa, Abscheren 100 000 Pa, 250 000 Pa, 400 000 Pa und 100 000 Pa) und aus den dabei bestimmten Messwerten der FFC-Wert ermittelt. Das Fliessverhalten lässt sich wie folgt charakterisieren:

| FFC | Fliessfähigkeit |
|-----|-----------------|
| >10 | frei fließend |
| 4-10 | leicht fließend |
| 2-4 | kohäsiv |
| 1-2 | sehr kohäsiv |
| <1 | nicht fließend |

**Beispiele**

Im folgenden wird die Erfindung anhand von Beispielen erläutert. Diese Erläuterungen sind lediglich beispielhaft und schränken den allgemeinen Erfindungsgedanken nicht ein. Sofern nicht anders angegeben, wiesen alle Polymerpulver eine Feuchtigkeit von unter 5 Gew.% auf, die Behandlungen mit den Salzlösungen wurden bei Raumtemperatur durchgeführt.

**Beispiel 1 - 5**

**Vergleichsbeispiel 1 - 4**

Aus einer großtechnischen Produktion wurde das Pulver eines an der Oberfläche nachvernetzten, superabsorbierenden, teilweise neutralisierten Acrylsäurepolymerisates entnommen und ohne vorherige Absiebung der Feinanteile (3 Gew.% unter 150 μm) mit wässrigen Lösungen von Aluminiumsulfat, Eisen(III)chlorid und Magnesiumchlorid unter Rühren mit einem Krups-Haushaltsmixer versetzt und auf einer Rollbank zum Abbau von Agglomeraten langsam durchmischt. Das Mengenverhältnis von Salz und Wasser sowie die Eigenschaften der mit den Salzlösungen behandelten Pulver sowie die des Originalpulvers ohne Absiebung (V1) bzw. nach Absiebung (V2) der Feinanteile unter 150 μm sind in der Tabelle 1 aufgeführt.

Tabelle 1:

| Zugabemengen Salz bzw. Wasser in Gew.% auf Polymerpulver: | | | | | |
|---|---|---|---|---|---|
| **Beispiel** | **$Al_2(SO_4)_3$\*/$H_2O$** | **$FeCl_3$+/$H_2O$** | **$MgCl_2$#/$H_2O$** | **AAP** | **TB** | **SFC** |
| **V1** | | | | 23,2 | 24 | 52 |
| **V2** | | | | 23,5 | 24 | 69 |
| **B1** | 0,1/0,5 | | | 22,5 | 24 | 53 |
| **B2** | 0,1/3,0 | | | 21,6 | 23 | 60 |
| **B3** | 1,0/1,0 | | | 21,1 | 24 | 105 |
| **B4** | | 0,25/0,5 | | 21,6 | 23 | 98 |
| **B5** | | 0,75/3,0 | | 20,6 | 23 | 120 |
| **V3** | | | 0,25/3,0 | 22,1 | 23 | 48 |
| **V4** | | | 0,25/1,0 | 22,3 | 23 | 37 |

\*: als Hydrat mit 18 $H_2O$,

+: als Hydrat mit 6 $H_2O$,

#: als Hydrat mit 6 $H_2O$

**Vergleichsbeispiel 5:**

Es wird wie bei V3 verfahren, jedoch findet $CaCl_2$ anstelle von $MgCl_2$ Verwendung. Das Polymerpulver wies eine Teebeutelretention von TB = 23 g/g, eine Absorption unter Druck von AAP = 21,9 g/g und eine Gelpermeabilität von SFC = 42 $[cm^3 * s * g^{-1}]$ auf.

**Beispiel 6:**

Ein mit einer Aluminiumsulfatlösung behandeltes oberflächenvernetztes Polymerpulver gemäß Beispiel 8 wird unter den Bedingungen des Anticakingtestes 3 h bei 38 °C und 80 % rel. Luftfeuchte gelagert und anschließend dem Siebtest unterzogen. Im Vergleich zu einer unbehandelten Probe passiert das erfindungsgemäße Polymerpulver fast quantitativ das Prüfsieb während die unbehandelte Probe aufgrund ihrer Verklumpungsneigung größtenteils auf dem Prüfsieb verbleibt.

Tabelle 2

| Produkt Behandlung | Feuchtigkeitszunahme [%] | Siebdurchgang [%] |
|---|---|---|
| ohne $Al_2(SO_4)_3$ | 10,2 | 33,0 |
| mit $Al_2(SO_4)_3$ | 8,1 | 99,8 |

**Beispiel 7:**

Das Pulver eines an der Oberfläche nachvernetzten Superabsorbers vom Typ Favor® SXM 9100[1] mit 3,9 Gew.% Feinanteil von unter 150 µm und einer Gelpermeabilität von SFC = 19 $[cm^3 * s * g^{-1}]$ wurde mit 1 Gew.% einer 50%igen $Al_2(SO_4)_3$ x14 $H_2O$-Lösung in einem Ruberg-Mischer homogen vermischt. Danach wies das Polymerpulver nur noch 2,0 Gew.% Feinanteile auf und die Gelpermeabilität war auf SFC = 50 $[cm^3 * s * g^{-1}]$ angestiegen.
Trennt man von dem nicht mit $Al_2(SO_4)_3$ —Lösung behandelten Ausgangspulver die Feinanteile unter 150 µm ab, so entsteht ein Pulver mit einem SFC = 43 $[cm^3 * s * g^{-1}]$.

**Beispiel 8:**

Entsprechend der Vorgehensweise von Beispiel 7 wird an der Oberfläche nachvernetztes Superabsorberpulver vom Typ Favor® SXM 6565[2] mit 3,1 Gew.% Feinanteil unter 150 µm mit unterschiedlichen Mengen 50 Gew.%iger $Al_2(SO_4)_3$ x14 $H_2O$-Lösung versetzt.
Zur Beurteilung der Abriebstabilität wurde die Permeabilität nach dem Mahlen einer Probe ohne und mit erfindungsgemäßer Behandlung gemessen. Der Mahltest wurde in einer Kugelmühle bei 95 UpM mit 10 g Produkt über 6 Minuten durchgeführt. Die Messung der Permeabilität erfolgt nach dem Mahltest ohne Abtrennung etwaiger Feinanteile.

Tabelle 3

| Gew.% $Al_2(SO_4)_3$ -Lösung | 0 | 0,4 | 1,0 | 2,0 | 4,0 |
|---|---|---|---|---|---|
| SFC ohne Abtrennung der Feinanteile unter 150 µm | 44 | 52 | 67 | 89 | 87 |
| SFC mit Abtrennung der Feinanteile unter 150 µm | 71 | | | | |
| SFC nach Kugelmühle | 20 | | | | 79 |

**Beispiel 9:**

Ein an der Oberfläche nachvernetztes, 3 Gew.% Feinanteile (<150 µm) enthaltendes Superabsorberpulver aus auf Polyvinylalkohol (Mowiol 5-88, 1,9 Gew.%/Trockensubstanz) gepfropfter, vorvernetzter und zu 70 Mol% teilneutralisierter Polyacrylsäure, wurde erfindungsgemäß mit 1 Gew.% einer 50%igen wässrigen Lösung von $Al_2$$(SO_4)_3$x18$H_2O$ durch Vermischen auf einer Rollbank behandelt. Das Produkt besaß eine Retention von 25,5 g/g, eine Absorption unter Druck von AAP = 21 g/g und eine Gelpermeabilität von SFC = 75 $[cm^3 * s * g^{-1}]$.
Ohne Behandlung wies der Superabsorber einschließlich von 3 Gew.% Feinanteilen (<150 µm) eine Teebeu-

[1] : Oberflächenvernetztes Superabsorberpulver aus vorvemetzter, teilneutralisierter Polyacrylsäure der Fa. Stockhausen GmbH&CO. KG, Krefeld, Deutschland
[2] : Oberflächenvernetztes Superabsorberpulver aus vorvemetzter, teilneutralisierter Polyacrylsäure der Fa. Stockhausen GmbH&CO. KG, Krefeld, Deutschland

telretention von TB = 25 g/g, eine Absorption unter Druck von AAP = 22 g/g und eine Gelperrneabilität von SFC = 45 [cm$^3$ * s * g$^{-1}$] auf. Nach dem Absieben der Feinanteile zeigte der unbehandelte Absorber bei gleichbleibender Retention eine Absorption unter Druck von AAP = 22,5 g/g und eine Gelpermeabilität von SFC = 75 [cm$^3$ * s * g$^{-1}$].

**Beispiel 10 - 13**

**Vergleichsbeispiel 6 - 7**

Ein Superabsorberpulver gemäss Beispiel 1 mit 3 Gew. Feinanteilen von <150 μm wird wie in Beispiel 1 behandelt. Die Eigenschaften der Absorberpulver mit Feinanteilen (V6), ohne Feinanteile (V7) und mit erfindungsgemäßer Nachbehandlung sind in der Tabelle 4 aufgeführt.

Tabelle 4

| Zugabemengen Salz bzw. Wasser in Gew.% auf Polymerpulver: | | | | | |
|---|---|---|---|---|---|
| **Beispiel** | **Al$_2$(SO$_4$)$_3$*/H$_2$O** | **FeCl$_3$$^+$/H$_2$O** | **AAP** | **TB** | **SFC** |
| **V6** | | | 23 | 24 | 45 |
| **V7** | | | 23,5 | 23,7 | 69 |
| **B10** | 0,5/0,5 | | 21,5 | 24,5 | 93 |
| **B11** | 1,0/3,0 | | 22 | 24 | 72 |
| **B12** | | 0,25/0,5 | 21 | 24 | 76 |
| **B13** | | 0,5/3,0 | 21 | 23,5 | 60 |

*: als Hydrat mit 18 H$_2$O,
$^+$: als Hydrat mit 6 H$_2$O

**Beispiel 14 - 19**

**Vergleichsbeispiele 8 - 10**

Ein Superabsorberpulver gemäss Beispiel 1 mit 5, 10 und 15 Gew.% Feinanteilen von<150 μm wird wie in Beispiel 1 behandelt. Die Eigenschaften der Absorberpulver mit Feinanteilen (V8-V10) und mit erfindungsgemäßer Nachbehandlung sind in der Tabelle 5 aufgeführt.

Tabelle 5

| Zugabemengen Salz bzw. Wasser in Gew.% auf Polymerpulver: | | | | | | |
|---|---|---|---|---|---|---|
| **Beispiel** | **Feinanteile vor Behandlung** | **Al$_2$(SO$_4$)$_3$*/H$_2$O** | **AAP** | **TB** | **SFC** | **Feinanteile nach Behandlung** |
| **V8** | 5 | | 22 | 24 | 26 | |
| **B14** | 5 | 0,5/0,5 | 21,3 | 24,7 | 52 | |
| **B15** | 5 | 0,5/3,0 | 20,2 | 24,4 | 45 | 2,5 |
| **V9** | 10 | | 21,4 | 24,3 | 29 | |
| **B16** | 10 | 0,5/0,5 | 20,8 | 24,3 | 53 | |
| **B17** | 10 | 0,5/3,0 | 20 | 23,7 | 45 | 3,5 |
| V10 | 15 | | 21,5 | 23,2 | 25 | |
| **B18** | 15 | 0,5/0,5 | 19,6 | 23,7 | 53 | |
| **B19** | 15 | 0,5/3,0 | 19,8 | 23,8 | 40 | 4,5 |

*: als Hydrat mit 18 H$_2$O

**Beispiel 20 -24**

**Vergleichbeispiel 11 - 15**

Pulver eines an der Oberfläche nachvernetzten, zu 70 Mol. % mit Natronlauge teilneutralisierten Polyacrylsäurepolymerisates mit einem Feinstaubanteil < 150 μm von 1,1 Gew.% wurden in einem MTI-Mischer (Schaufelmischer) mit verschieden konzentrierten wässrigen Lösungen von Aluminiumsulfat versetzt und bei 750 Upm durchmischt.

Tabelle 6

| Zugabemengen Salz bzw. Wasser in Gew.% auf Polymerpulver: | | | | | |
|---|---|---|---|---|---|
| **Beispiel** | **$Al_2(SO_4)_3$\*/$H_2O$** | **FFC** | **AAP** | **TB** | **SFC** |
| **B20** | 0,15/0,15 | 14 | 22, 5 | 27 | 47 |
| **V11** | ohne | 5,7 | 23 | 26,5 | 44 |
| **B21** | 0,15/0,5 | 11 | 22,8 | 26 | 41 |
| **V12** | ohne | 6,2 | 23,3 | 26,7 | 37 |
| **B22** | 0,15/1,0 | 10,4 | 22,8 | 26,5 | 48 |
| **V13** | ohne | 6,2 | 23,5 | 27 | 37 |
| B23 | 0,15/2,0 | 8,6 | 22,3 | 26,8 | 45 |
| **V14** | ohne | 6,2 | 24 | 26,3 | 38 |
| **B24** | 0,15/3,0 | 8,3 | 22 | 26 | 63 |
| **V15** | ohne | 6,8 | 23,8 | 26,5 | 46 |

\*: als Hydrat mit 18 $H_2O$

Die Produkte wiesen eine gute bis sehr gute Fliessfähigkeit, die bei den Beispielen 20-22 sofort nach kurzer Durchmischung vorhanden war, während die Produkte nach Beispiel 23 und 24, die mit Salzlösungen geringerer Konzentration behandelt wurden, eine längere Stand- bzw. Mischzeit von bis zu 1 Stunde erforderten.

**Patentansprüche**

1. Pulverförmiges, an der Oberfläche nachvernetztes, Wasser oder wässrige Flüssigkeiten absorbierendes Polymerisat, aufgebaut aus polymerisierten gegebenenfalls vorvernetzten, teilneutralisierten Carboxylgruppen enthaltenden Monomeren, **dadurch gekennzeichnet, dass** das pulverförmige Polymerisat nach der Nachvemetzung mit der Lösung wenigstens eines Salzes eines mindestens dreiwertigen Kations umgesetzt worden ist.

2. Pulverförmiges Polymerisat nach Anspruch 1, **dadurch gekennzeichnet, dass** das Kation mindestens ein Aluminium-Kation, Eisen-Kation und/oder Mangan-Kation ist.

3. Pulverförmiges Polymerisat nach Anspruch 1 oder 2, **dadurch gekennzeichnet, dass** das Kation in einer Menge von 0,001 - 1 Gew.-%, vorzugsweise mit 0,002 - 0,5, besonders bevorzugt 0,005 - 0,2 Gew.-% bezogen auf das Polymerisat eingesetzt wird.

4. Pulverförmiges Polymerisat nach einem der Ansprüche 1 - 3, **dadurch gekennzeichnet, dass** die Carboxylgruppen zu 25 - 85 Mol% neutralisiert sind.

5. Pulverförmiges Polymerisat nach einem der Ansprüche 1 - 4, **dadurch gekennzeichnet, dass** es weitere Comonomere und/oder Pfropfpolymere enthält.

6. Verfahren zur Herstellung von pulverförmigem, an der Oberfläche nachvernetztem, Wasser oder wässrige Flüssigkeiten absorbierenden Polymerisat gemäß einem der Ansprüche 1 - 5, **dadurch gekennzeichnet, dass** dem pulverförmigen Polymerisat nach der Nachvernetzung eine Lösung wenigstens eines Salzes eines mindestens dreiwertigen Kations zugesetzt wird und das pulverförmige Polymerisat und die Lösung vorzugsweise homogen durchmischt werden.

7. Verfahren nach Anspruch 6, **dadurch gekennzeichnet, dass** die Temperatur des Polymerisatpulvers während der Nachbehandlung 0°C bis 100°C, vorzugsweise 10°C bis 80°C, besonders bevorzugt 20 bis 50°C beträgt.

8. Pulverförmiges, Wasser oder wässrige Flüssigkeiten absorbierendes Polymerisat, erhältlich durch Verfahren gemäss einem der Ansprüche 6 oder 7.

9. Verwendung des pulverförmigen Polymerisates gemäss einem der Ansprüche 1-5 und 8 als Absorptionsmittel für Flüssigkeiten, insbesondere Wasser und wässrige Flüssigkeiten.

10. Verwendung einer Lösung wenigstens eines Salzes eines mindestens dreiwertigen Kations zur Wiederherstellung der Gelpermeabilität von durch mechanische Einwirkung geschädigten pulverförmigen Wasser oder wässrige Flüssigkeiten absorbierenden Polymerisaten, aufgebaut aus polymerisierten gegebenenfalls vorvernetzten, teilneutralisierten Carboxylgruppen enthaltenden Monomeren, **dadurch gekennzeichnet, dass** dem pulverförmigen Polymerisat nach der Nachvemetzung die Lösung des Salzes zugesetzt wird und das pulverförmige Polymerisat und die Lösung durchmischt werden.

11. Verwendung des Polymerisates gemäss den Ansprüchen 1 - 4 oder hergestellt mit dem Verfahren gemäss den Ansprüchen 6 oder 7 als Absorptionsmittel für wässrige Flüssigkeiten, vorzugsweise in Konstruktionen zur Aufnahme von Körperflüssigkeiten, in geschäumten und nicht geschäumten Flächengebilden, in Verpackungsmaterialien, bei der Pflanzenaufzucht und als Bodenverbesserungsmittel; oder in Hygieneartikeln, insbesondere Windeln oder Tampons; oder als Trägersubstanz und/oder Stabilisator für Wirkstoffe, insbesondere für Düngemittel oder andere Wirkstoffe, die gegebenenfalls retardiert abgegeben werden.

12. Hygiene artikel, insbesondere Windeln aufweisend Polymerisate nach einem der Ansprüche 1 bis 4 oder hergestellt mit dem Verfahren gemäss den Ansprüchen 6 oder 7.

## Claims

1. Pulverulent polymerizate which has been post-crosslinked at the surface, absorbs water or aqueous fluids and is composed of polymerised, optionally pre-crosslinked monomers containing partially neutralised carboxyl groups, **characterised in that** the pulverulent polymerizate has been reacted following the post-crosslinking with a solution of at least one salt of an at least trivalent cation.

2. Pulverulent polymerizate according to claim 1, **characterised in that** the cation is at least one aluminium cation, iron cation and/or manganese cation.

3. Pulverulent polymerizate according to claim 1 or 2, **characterised in that** the cation is used in an amount of from 0.001 to 1 % by weight, preferably from 0.002 to 0.5 % by weight, especially from 0.005 to 0.2 % by weight, based on the polymerizate.

4. Pulverulent polymerizate according to any one of claims 1 to 3, **characterised in that** the carboxyl groups are neutralised to the extent of from 25 to 85 mol.%.

5. Pulverulent polymerizate according to any one of claims 1 to 4, **characterised in that** it comprises further comonomers and/or graft polymers.

6. Process for the preparation of a pulverulent polymerizate which has. been post-crosslinked at the surface and absorbs water or aqueous fluids according to any one of claims 1 to 5, **characterised in that** a solution of at least one salt of an at least trivalent cation is added to the pulverulent polymerizate following the post-crosslinking, and the pulverulent polymerizate and the solution are mixed thoroughly, preferably homogeneously.

7. Process according to claim 6, **characterised in that** the temperature of the powdered polymerizate during the after-treatment is from 0°C to 100°C, preferably from 10°C to 80°C, especially from 20 to 50°C.

8. Pulverulent polymerizate which absorbs water or aqueous fluids, obtainable by processes according to either claim 6 or claim 7.

9. Use of the pulverulent polymerizate according to any one of claims 1 to 5 and 8 as an absorbent for fluids, especially water and aqueous fluids.

10. Use of a solution of at least one salt of an at least trivalent cation to restore the gel permeability of pulverulent polymerizates which absorb water or aqueous fluids and are composed of polymerised, optionally pre-crosslinked monomers containing partially neutralised carboxyl groups, when such polymerizates have been damaged by mechanical action, **characterised in that** the solution of the salt is added to the pulverulent polymerizate following the post-crosslinking, and the pulverulent polymerizate and the solution are mixed thoroughly.

11. Use of the polymerizate according to claims 1 to 4 or prepared by means of the process according to claim 6 or 7 as an absorbent for aqueous fluids, preferably in structures for absorbing body fluids, in foamed and non-foamed sheet-like structures, in packaging materials, in horticulture and as a soil improver; or in hygiene articles, especially diapers or tampons; or as a carrier and/or stabiliser for active ingredients, especially for fertilisers or other active ingredients, which are optionally released in a delayed manner.

12. Hygiene articles, especially diapers, comprising polymerizates according to any one of claims 1 to 4 or prepared using the process according to claim 6 or 7.

**Revendications**

1. Polymère en poudre, post-réticulé en surface, absorbant l'eau ou les liquides aqueux, constitué de monomères contenant des groupes carboxyle partiellement neutralisés, éventuellement pré-réticulés, polymérisés, **caractérisé en ce que** le polymère en poudre est mis en réaction, après la post-réticulation, avec la solution d'au moins un sel d'un cation au moins trivalent.

2. Polymère en poudre selon la revendication 1, **caractérisé en ce que** le cation est au moins un cation aluminium, un cation fer et/ou un cation manganèse.

3. Polymère en poudre selon la revendication 1 ou 2, **caractérisé en ce que** l'on utilise une quantité de cation de 0,001 à 1 % en poids, de préférence de 0,002 à 0,5 %, plus préférablement de 0,005 à 0,2 % en poids par rapport au polymère.

4. Polymère en poudre selon l'une quelconque des revendications 1 à 3, **caractérisé en ce que** les groupes carboxyle sont neutralisés à 25 à 85 % en moles.

5. Polymère en poudre selon l'une quelconque des revendications 1 à 4, caractérisé en qu'il contient d'autres comonomères et/ou polymères greffés.

6. Procédé de fabrication d'un polymère en poudre, post-réticulé en surface, absorbant l'eau ou les liquides aqueux selon l'une quelconque des revendications 1 à 5, **caractérisé en ce que** l'on ajoute au polymère en poudre, après la post-réticulation, une solution d'au moins un sel d'un cation au moins trivalent et on mélange intimement de préférence de manière homogène le polymère en poudre et la solution.

7. Procédé selon la revendication 6, **caractérisé en ce que** la température de la poudre de polymère est comprise, pendant le post-traitement, entre 0° C et 100° C, de préférence entre 10° C et 80° C, plus préférablement entre 20 et 50° C.

8. Polymère en poudre absorbant l'eau ou les liquides aqueux pouvant être obtenu selon l'une quelconque des revendications 6 ou 7.

9. Utilisation du polymère en poudre selon l'une quelconque des revendications 1 à 5 et 8 en tant qu'agent d'absorption pour des liquides, en particulier l'eau et les liquides aqueux.

10. Utilisation d'une solution d'au moins un sel d'un cation au moins trivalent pour le rétablissement de la perméabilité de gel de polymères en poudre absorbant l'eau ou des liquides aqueux, détériorés par une action mécanique, constitués de monomères contenant des groupes carboxyle partiellement neutralisés, éventuellement pré-réticulés, polymérisés, **caractérisée en ce que** l'on ajoute au polymère en poudre, après la post-réticulation, la solution du sel et on mélange intimement le polymère en poudre et la solution.

11. Utilisation du polymère selon les revendications 1 à 4 ou fabriqué avec le procédé selon les revendications 6 ou 7 en tant qu'agent d'absorption pour liquides aqueux, de préférence dans des constructions destinées à recevoir des liquides corporels, dans des produits plans moussés et non moussés, dans des matériaux d'emballage, dans la culture des plantes et en tant qu'agent d'amendement des sols ; ou bien dans des articles d'hygiène, en particulier dans les couches et les tampons ; ou bien en tant que substance support et/ou stabilisant pour substances actives, en particulier pour des engrais ou autres substances actives qui sont éventuellement à libération retardée.

12. Articles d'hygiène, en particulier couches présentant des polymères selon l'une quelconque des revendications 1 à 4 ou fabriqués avec le procédé selon les revendications 6 ou 7.